# EUROPEAN PATENT APPLICATION

(11) **EP 4 563 105 A2**
(43) Date of publication of application: **04.06.2025**
(21) Application number: 24216039.8
(22) Date of filing: 28.11.2024
(51) Int. Cl.: A61B 17/72

(54) **TELESCOPIC INTRAMEDULLARY NAIL AND KIT COMPRISING SAID TELESCOPIC INTRAMEDULLARY NAIL AND CORRESPONDING TOOLS**

(30) Priority: 30.11.2023 IT 202300025602
(71) Applicant: Orthofix S.r.l., 37012 Bussolengo (VR) (IT)
(72) Inventor: VENTURINI, Daniele, 37064 Povegliano Veronese (VR) (IT); MAGNI, Marco, 44122 Ferrara (IT)
(74) Representative: Botti & Ferrari S.p.A.

(57) **Abstract**

A telescopic intramedullary nail (1) for the treatment of fractures or deformations of long bones, such as for example in the case of osteogenesis imperfecta or pseudoarthrosis, comprising:
a hollow stem (2); and
a rod (3) telescopically coupled inside said hollow stem (2);
wherein said hollow stem (2) comprises, at a free distal end (11), an anchor tip (21) configured to be internally fixed inside a distal portion of a long bone of a patient, without protruding from said long bone;
wherein said rod (3) comprises a free proximal end arranged to be fixed to a proximal site of said long bone.

## Description

### Field of the invention

The present invention relates to a nail which can be inserted into the long bones of a patient, preferably femur, tibia or humerus, and a kit comprising, in addition to the nail, the tools needed for the implant and the extraction of the different nail components.

In particular, the invention relates to a telescopic intramedullary nail intended for the treatment of fractures or deformations of long bones, such as for example in the case of osteogenesis imperfecta or pseudoarthrosis, in particular in childhood.

The telescopic intramedullary nail according to the invention is preferably intended to be implanted with an anterograde approach.

The invention is usefully applied in the field of paediatric orthopedics, for example for interventions to correct bone deformities of long bones due to osteogenesis imperfecta or congenital pseudoarthrosis. Therefore, the following description is made for use in the context of this field but it is not to be understood as limiting.

### Prior art

Known techniques for correcting long bone deformations in developmental-age patients affected by osteogenesis imperfecta provide to make at least one osteotomy, the alignment of bone fragments and the insertion of a nail into the medullary cavity or other cavity obtained along the bone.

The nails used in this type of interventions are of the telescopic type, that is they provide a hollow stem and a rod which is fixable and slidingly inserted into said hollow stem, the ends of the hollow stem and of the rod being constrained to the two opposite ends of the long bone, respectively. The relative sliding of the two components during the bone growth allows the nail to adapt to the length variation of the bone itself ensuring that the alignment is maintained.

Telescopic intramedullary nails of the Fassier-Duval type, which adopt an anterograde surgical approach, are widely used in the prior art. In fact, the anterograde approach is almost mandatory in tibia applications, where an access from the ankle joint has high risks of damaging the joint itself, and in many cases it is preferred in femur applications as well to avoid entering from the knee joint.

In the above Fassier-Duval nail it is provided that the rod has a threaded tip arranged to be screwed into a distal epiphysis of the long bone, while the hollow stem inside which the rod slides has a threaded head which can be screwed into the proximal epiphysis with respect to the operation point.

The threaded tip is thus fixed beyond the growth cartilage. The rod, having the tip fixed to the distal end of the bone, moves inside the hollow stem during growing, so that the nail adapts to the progressive lengthening of the bone.

The surgical technique for implanting the Fassier-Duval nail provides, once the osteotomies have been made and the bone fragments have been straightened, the creation of a cavity inside the bone by means of a perforator, guided by a Kirchner wire, which enlarges the medullary cavity for housing the nail components.

Once the cavity has been created, the perforator is removed.

The rod is then inserted into the cavity at an end of the bone up to screw the threaded tip into the epiphysis of the opposite end. The proximal end of the rod is then cut to measure directly on site.

The hollow stem is then inserted into the cavity so as to slidingly house the rod and the threaded head screwed into the bone.

The insertion of the nail components is performed using tools coupled to the end of the component which provides a handle to support the insertion and advance of the nail into the cavity.

The perforator used in these surgical operations provides a perforation end shaped to remove bone during the rotational advance.

Although advantageous in many respects, the Fassier-Duval nails have several drawbacks which are still unsolved to date.

A first drawback relates to the difficulty in extracting the rod component at the time of removing the nail. For the removal it is first necessary to grip this component, which is externally smooth and has a relatively small diameter, inside the medullary cavity; it is thus necessary to perform a torsion in order to unscrew the thread from the epiphysis; finally a traction is to be performed in order to extract the component from the bone. These operations are in most cases extremely complex for the surgeon.

A second drawback derives from the loss of continuity of the bone axis during the insertion of the nail. In fact, the rod, which is the first component being inserted, is not cannulated and the Kirchner wire used to bore the medullary cavity must be removed before the insertion thereof.

A third drawback still relates to possible seizure problems, due to the local deformation of the rod in the point in which it is cut to measure. The rod may thus hardly enter and slide inside the hollow stem.

Finally, a fourth drawback derives from the selection of the correct size of the hollow stem to be used after the insertion of the rod.

The technical problem underlying the present invention is to solve or at least mitigate the drawbacks mentioned with reference to the Fassier-Duval nail.

A particular object of the present invention is to devise a telescopic intramedullary nail and a corresponding kit with tools allowing an easy extraction of both components from the bone site.

A further particular object of the present invention is to devise a telescopic intramedullary nail which avoids the loss of continuity of the bone axis during the insertion.

A further particular object of the present invention is to devise a telescopic intramedullary nail which allows to avoid the cut in site of the rod, avoiding the drawbacks of device seizure at the cut.

A further object is to devise a nail and a corresponding insertion kit which facilitates the size selection of the components at the time of implantation.

### Summary of the invention

The above objects are achieved by a telescopic intramedullary nail for the treatment of fractures or deformations of long bones, such as for example in the case of osteogenesis imperfecta or pseudoarthrosis, comprising:
a hollow stem; and
a rod telescopically coupled inside said hollow stem;
wherein said hollow stem comprises, at a free distal end, an anchor tip configured to be internally fixed inside a distal portion of a long bone of a patient, preferably in the epiphysis, without protruding from said long bone;
wherein said rod comprises a free proximal end arranged to be fixed to a proximal site of said long bone.

The idea underlying the present invention is to reverse the insertion sequence, inserting first the female part, that is the hollow stem, which is suitable for cannulation and allows an easier grip in the removal step; and then inserting the male part, that is the rod.

Preferably said rod comprises, at the proximal end, a terminal head arranged to externally abut against a proximal cortical of said long bone.

The terminal head can be integrally formed with the rest of the rod, since the rod will be inserted from the insertion hole against which said head abuts.

The rod can comprise, in a position which is adjacent to the terminal head, a cylindrical portion with a diameter which is greater than an internal diameter of the hollow stem, which ensures a greater stability to the rod in the proximal section.

As an alternative, the terminal head can comprise a conical thread which connects to a cylindrical thread provided on a first proximal section of the rod.

The hollow stem can advantageously comprise, at a proximal end thereof, coupling means for an insertion tool and/or for an extraction tool of said hollow stem.

Said coupling means are preferably arranged at an insertion mouth of the rod and comprise an internal thread in said insertion mouth.

Said coupling means can further comprise an end crenelation made frontally to said insertion mouth.

In a preferred embodiment, the anchor tip has a thread for the screwing in the epiphysis.

Advantageously, the screwing direction of the thread of the anchor tip can be opposite to that of the internal thread of the coupling means of the hollow stem, so that a removal tool can couple to the internal thread by screwing and, by continuing the rotation, unscrew the hollow stem from the seat thereof in the epiphysis.

In an alternative embodiment, the anchor tip is not threaded but has a transverse through hole for the insertion of an anchor pin. This alternative allows the fixing of the telescopic intramedullary nail if the epiphysis is not extended enough for thread anchoring.

Advantageously said anchor tip can be cannulated so that the Kirchner wire has not to be extracted before the insertion of the first component of the telescopic intramedullary nail, ensuring the continuity of the bone axis.

The nail according to the invention is preferably an anterograde nail, i.e. intended to be implanted with a preferably anterograde surgical approach.

The above-identified technical problem is also solved by a kit comprising a telescopic intramedullary nail of the above-identified type and at least one removal tool comprising a stem and a boring portion arranged to be coupled to coupling means arranged at an end of the hollow stem. In particular, the boring portion can comprise a threaded tip and/or a front toothing, arranged to be coupled to the internal thread and to the end crenelation of the coupling means.

The above-identified technical problem is also solved by a kit comprising a telescopic intramedullary nail of the above-described type and a drill bit comprising a stem provided with progressive length indexes in order to identify during a boring step the length of the telescopic intramedullary nail components to be implanted.

The above-identified technical problem is also solved by a kit comprising a telescopic intramedullary nail of the above-described type; a left-handed tension rod comprising a stem provided with a left-handed threaded tip; and a right-handed tension rod comprising a stem provided with a right-handed threaded tip. Those tension rods can be alternatively coupled with a sleeve provided with front engaging means for the respective insertion of the rod and of the hollow stem.

Further features and advantages of the telescopic intramedullary nail and corresponding kit according to the present invention will be more apparent from the following description of exemplary embodiments given by way of non-limiting examples.

### Brief description of the drawings

The present description refers to the attached drawings, in which:

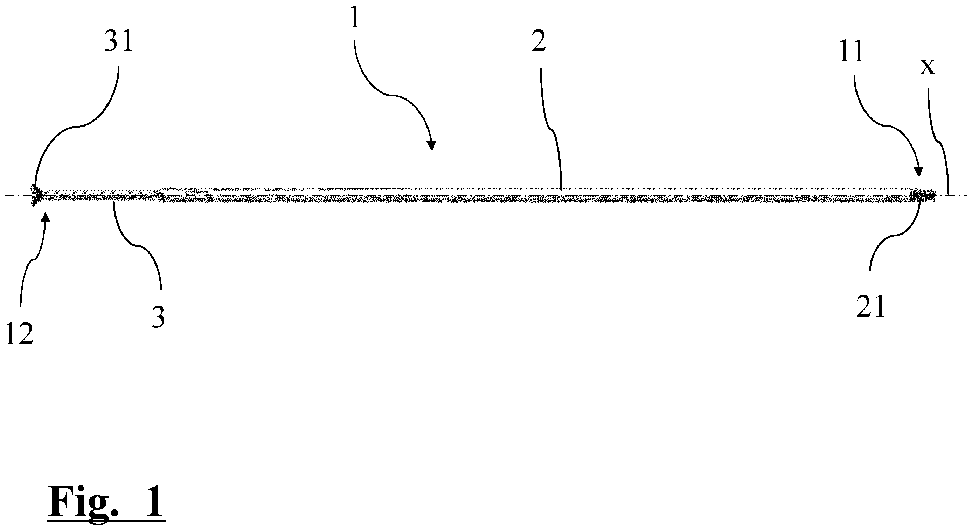

- Figure 1 shows a side view of an embodiment of an assembled telescopic intramedullary nail made in accordance with the present invention;
- Figure 2a shows a perspective view of an anchor tip of the hollow stem of the telescopic intramedullary nail in accordance with a first alternative embodiment;
- Figure 2b shows a perspective view of an anchor tip of the hollow stem of the telescopic intramedullary nail in accordance with a second alternative embodiment;

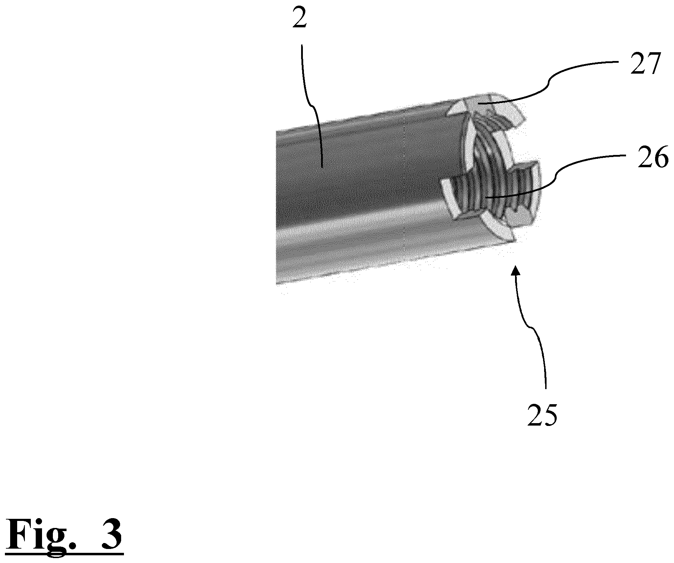

- Figure 3 shows a perspective view of a proximal anchoring end of the hollow stem of the telescopic intramedullary nail of Figure 1;
- Figure 4a shows a perspective view of a proximal end of the rod of the telescopic intramedullary nail in accordance with a first alternative embodiment;
- Figure 4b shows a perspective view of a proximal end of the rod of the telescopic intramedullary nail in accordance with a first alternative embodiment;

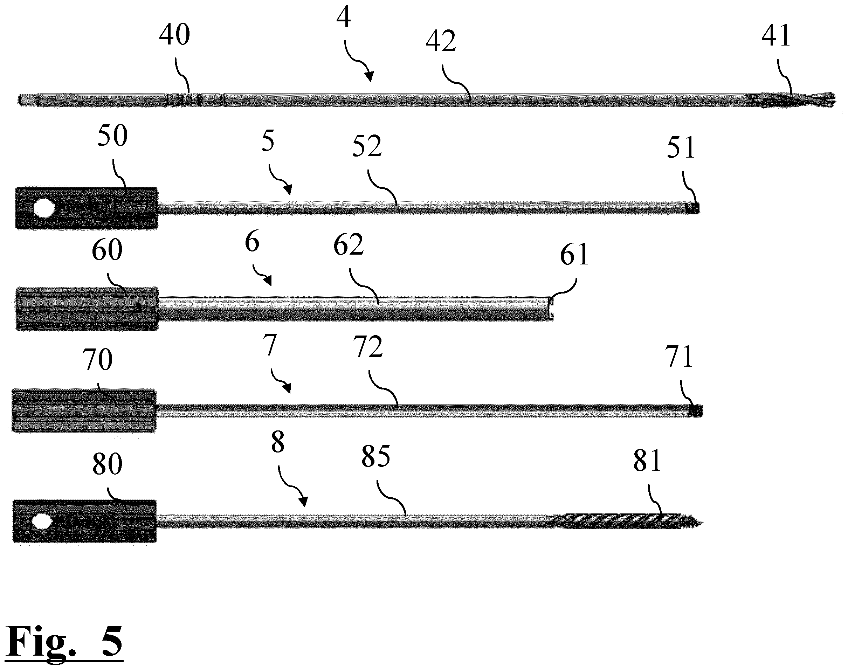

- Figure 5 shows tools used for the insertion and removal of the telescopic intramedullary nail according to the present invention;
- Figure 6 shows an enlarged detail of a removal tool which is visible in Figure 5;
- Figure 7 shows an enlarged detail of a drill bit which is visible in Figure 5;
- Figures 8-12 show the successive steps of a method for inserting a telescopic intramedullary nail according to the present invention into the femur of a patient;
- Figure 13 shows the telescopic intramedullary nail according to the present invention implanted into the femur of a patient;
- Figures 14 and 15 show the successive steps of a method for removing a telescopic intramedullary nail according to the present invention from the femur of a patient.

In different figures, similar elements will be indicated with similar reference numbers.

### Detailed description of the preferred embodiments

With reference to the attached figures, illustrative but non-limiting embodiments of the anterograde telescopic intramedullary nail 1 according to the present invention are described below.

The telescopic intramedullary nail according to the invention is particularly suitable for the treatment of fractures or deformations of long bones 100, such as for example in the case of osteogenesis imperfecta or pseudoarthrosis, in particular in paediatric patients.

The telescopic intramedullary nail 1 generally comprises a hollow stem 2 and a rod 3, the rod 3 being telescopically inserted into said hollow stem 2. Said rod 3 is thus slidingly coupled inside the hollow stem 2.

In the embodiment illustrated in the figure the telescopic coupling provides an external cylindrical surface of the rod 3 which is inserted into an internal cylindrical surface of the hollow stem 2, having a corresponding diameter net of the clearances to allow the telescopic sliding.

The use of cylindrical surfaces, although not having to be understood in a limiting sense, allows to minimize friction forces during the relative sliding between the two components of the telescopic intramedullary nail 1. This geometry is thus less subject to seizure problems if the telescopic intramedullary nail 1 is subjected to external stresses, in particular bending.

The mechanical features, and in particular the bending strength, can be sized for the same external diameter so that the strength of the hollow stem 2 and of the rod 3, when not overlapping, are equivalent or the one greater than the other when it is desired to adapt the mechanical strength of the nail to different stresses along the extension of the long bone.

The telescopic intramedullary nail 1 develops along a longitudinal axis X and is delimited by two ends, a distal end 11 of the hollow stem 2 and a proximal end 12 of the rod 3.

It is noted here that the terms terminal and distal refer to a preferred proximal-distal insertion of the telescopic intramedullary nail 1 into the long bone 100 of a patient, preferably a femur, a tibia or a humerus. On the other hand, the possibility that the telescopic intramedullary nail 1 can be implanted following an opposite orientation is not excluded.

The hollow stem 2 of the telescopic intramedullary nail 1 provides, at the distal end 11, an anchor tip 21, 2 1' arranged to be internally fixed in the epiphysis 102 of a long bone of the patient.

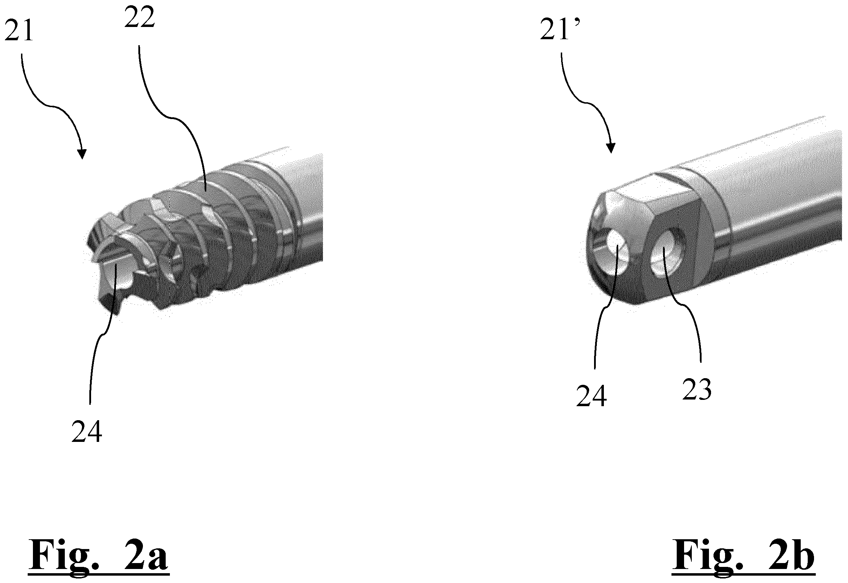

In a first embodiment, illustrated in detail in figure 2a, the anchor tip 21 has a self-threading thread 22, in particular a right-handed thread, arranged to screw into the bone epiphysis, according to modes described below. Said thread 22 ensures a traction stability and an easy penetration into the bone. The fixing of the anchor tip 21 is thus performed in this case with modes which are similar to those of the Fassier-Duval nail, described above with reference to the prior art.

In an alternative embodiment, the anchor tip 21' is not threaded and has instead a transverse through hole 23, into which an anchor pin not illustrated in the attached figures can be inserted. This second anchor tip 21' can be used as an alternative to the first one if the size of the epiphysis 102 of the patient does not allow to screw a threaded tip. Accordingly, the longitudinal dimension of the anchor tip 21' in accordance with this embodiment is smaller than that of the anchor tip 21 according to the first embodiment.

Both anchor tips 21, 21' are cannulated, that is they comprise a longitudinal channel 24, having a smaller diameter than that of the main body of the hollow stem 2, which allows the sliding above a Kirchner wire to guide the component in the insertion step.

The hollow stem 2 comprises, at the opposite end with respect to the anchor tip 21', that is at an insertion mouth of the rod 3, coupling means 25 for an insertion tool 5, 6 and for an extraction tool 8 of the component, better described below. Said coupling means 25 comprise an internal thread 26, in particular a left-handed internal thread, made on the internal cylindrical surface of the hollow stem 2. Moreover, said coupling means 25 comprise a front end crenelation 27, comprising projecting portions interspersed with offsets, made on the insertion mouth of the rod 3. Said end crenelation 27 is defined in particular by four equally spaced offsets, that is opposed two by two, which form therefore two diametrical grooves whose function will be described below.

The rod 3 of the telescopic intramedullary nail 1 provides, at the proximal end 12, a terminal head 31 arranged to externally abut against a proximal end of the long bone 100, lying therefore at least partially outside said bone.

Said terminal head 31 comprises an external conical thread 32 which screws in place into the hole of the proximal entry made on the long bone 100. The conical thread 32 can be interrupted by an anti-unscrewing groove, provided to increase the stability of the fixing to the bone. Advantageously, the conical thread 32 has such a high conical development as to ensure a high grip in a reduced bone thickness.

Moreover, the external face of said fixing head 31 has coupling means 33 for an insertion and extraction tool 6, 7, which are embodied in particular by a diametrical groove 34 and by an internally threaded central slot 35, in particular with a right-handed thread.

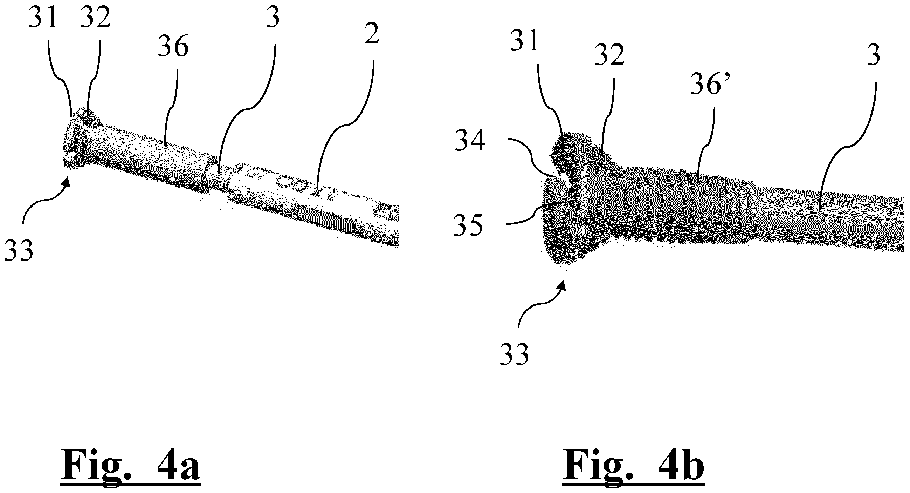

In an embodiment, illustrated in figure 4a, the terminal head 31 connects to the rest of the rod 3 by a cylindrical portion 36 with a greater diameter, which is approximately equal to that of the hollow stem 2, to ensure the stability of the proximal end 12.

In an alternative embodiment, illustrated in figure 4b, the terminal head 31 connects to the rest of the rod by a cylindrical thread 36', with the worm in phase with the conical thread 32 and with the same pitch, intended to thread into the hole at the proximal entry of the long bone 100.

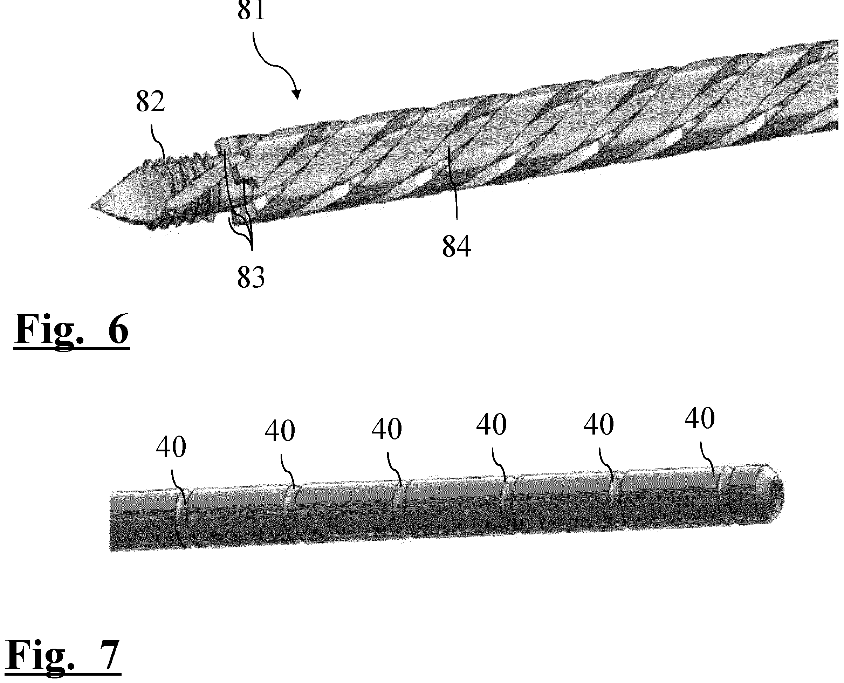

The tools for inserting and extracting the telescopic intramedullary nail 1 into the long bone 100 of a patient are illustrated in figure 6.

These tools comprise a drill bit 4, a left-handed tension rod 5, an engaging sleeve 6, a right-handed tension rod 7 and a removal tool 8.

The drill bit 4 has a cannulated stem 42 provided with a bone perforation end 41. In a spaced position with respect to the perforation end 41 there are progressive length indexes 40, whose function will be better described below. As it is better visible in figure 7, the length indexes can be successive notches formed on the external surface of the stem 42.

The left-handed tension rod 5 has a stem 52 provided with a threaded tip 51, in particular with a left-handed thread, at the distal end. This threaded tip 51 is arranged to screw into the internal thread 26 of the coupling means 25 of the hollow stem 2. At the proximal end there is instead a cylindrical handle 50 to allow the rotation of the stem 52 along its own axis.

The right-handed tension rod 7 has a stem 72 provided with a threaded tip 71, in particular with a right-handed thread, at the distal end. This threaded tip 71 is arranged to screw into the centrale slot 35 of the coupling means 33 of the rod 3. At the proximal end there is instead a cylindrical handle 70 to allow the rotation of the stem 72 along its own axis. The handle 70 is preferably made so as to be visually and/or tactilely distinguished from the handle 50 of the left-handed tension rod. The two handles can be of different colours, for example the handle 50 of the left-handed tension rod 5 can be red and the handle 70 of the right-handed tension rod 7 can be green.

The engaging sleeve 6 has a stem 62 provided at the distal end thereof with a front toothing 61, consisting in particular of two radial teeth aligned along a same diameter. Said front toothing 61 is arranged to engage into the end crenelation 27 of the coupling means 25 of the hollow stem 2; or into the diametrical groove 34 of the coupling means 33 of the rod 3. At the proximal end the engaging sleeve 6 has a cylindrical handle 60 to allow the rotation of the stem 52 along its own axis. The engaging sleeve 6 has a central cavity, which is not visible in the figures, to allow the rotatable housing of the stem 52 of the left-handed tension rod 5 or of the stem 72 of the right-handed tension rod 7.

The removal tool 8 is a manual borer comprising a stem 85 and a distal boring portion 81. As it is better visible in figure 6, the boring portion 81 comprises a threaded tip 82, in particular with a left-handed thread, and an adjacent stem portion provided with helical grooves 84. The portion with helical grooves 84 frontally faces the threaded tip 82, defining a front toothing 83, consisting in particular of four equally spaced teeth, arranged to abut against the end crenelation 27 of the coupling means 25 of the hollow stem 2. The threaded tip 82 is instead arranged to screw into the internal thread 26 of the coupling means 25 of the hollow stem 2. The removal tool 8 comprises, at the proximal end, a cylindrical handle 80 to allow the rotation of the stem 82 along its own axis. The handle 80 is preferably made so as to be visually and/or tactilely distinguished from the handle 70 of the right-handed tension rod 7, being instead similar to the handle 50 of the left-handed tension rod 5. The handle 80 of the removal tool 8 can be for example red like that of the left-handed tension rod 5.

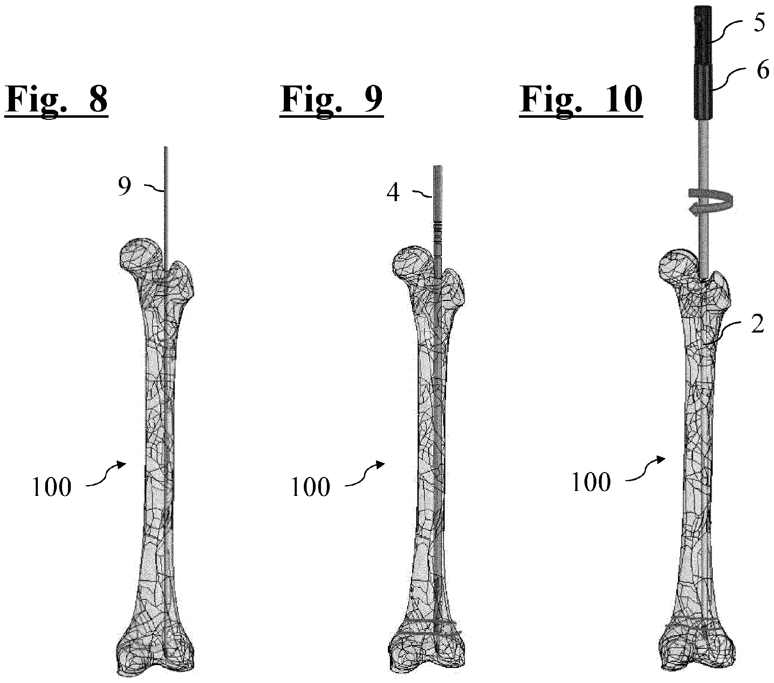

With reference to figures 8-13, a procedure for inserting the telescopic intramedullary nail 1 is now described.

In a first step, once possible osteotomies have been made and the bone fragments have been straightened, a Kirchner wire 9 is inserted through the medullary cavity of a long bone 100 of a patient. The Kirchner wire is inserted up to pass the growth cartilage 101 but without entering the joint. The step is illustrated in figure 8 with particular reference to a femur.

Once the Kirchner wire 9 has been fixed, the latter is used to guide the drill bit 4 which will bore the medullary cavity for the later insertion of the telescopic intramedullary nail 1. The drill bit 4 perforates passing the growing cartilage by a length comprised between 1 and 2 mm. Advantageously, due to the progressive length indexes 40, the boring step also allows an estimate of the length of the components of a telescopic intramedullary nail 1 to be used, thus avoiding the surgeon having to cut the components during the intervention. The boring step is illustrated in figure 9.

Once the bore has been made, the drill bit 4 is removed keeping the Kirchner wire 9 in place, which is used to guide the hollow stem 2 in the medullary cavity. The hollow stem 2 is inserted by means of an insertion tool 5, 6 defined by the insertion of the left-handed tension rod 5 into the engaging sleeve 6, according to the above-described coupling modes. For the last part of the insertion the tension rod 5 is removed, continuing the insertion with the engaging sleeve 6.

By means of the insertion tool 6, the distal anchor tip 21 is screwed down to the cancellous bone and the growth cartilage. Where the space is not enough for thread anchoring, the non-threaded anchor tip 21' described with reference to figure 2b is used. The above step of fixing the hollow stem 2 is illustrated in figure 10.

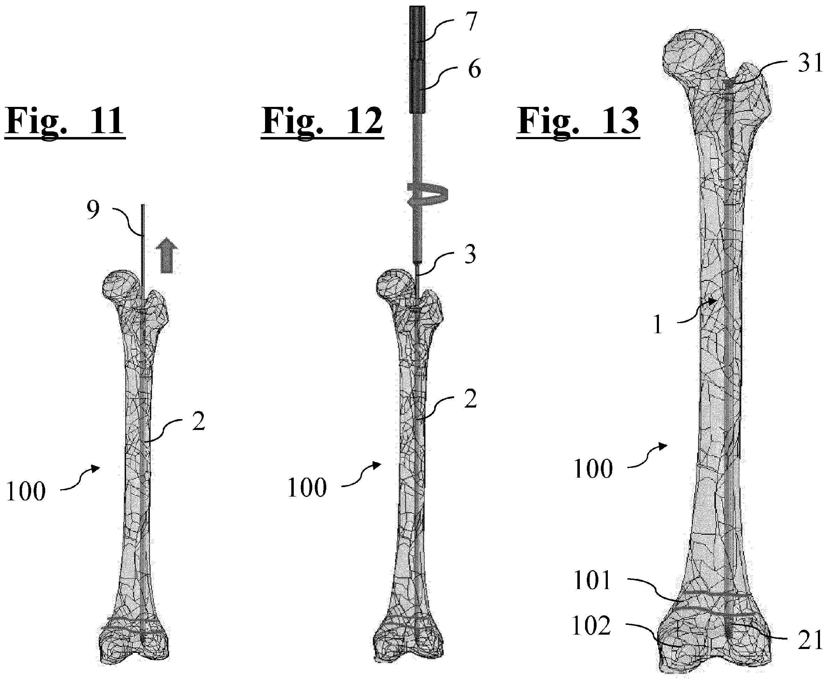

After fixing the hollow stem 2, the Kirchner wire 9 is removed as illustrated in figure 11.

In a following step the rod 3 is then inserted into the hollow stem 2. The rod 3 is inserted by means of an insertion tool 6, 7 defined by the insertion of the right-handed tension rod 7 into the engaging sleeve 6, according to the above-described coupling modes. By means of said insertion tool the rod 3 is rotated so as to screw the conical thread 32 of the terminal head 31 into the proximal cortical of the long bone 100. This last step of the insertion of the telescopic intramedullary nail 1 is shown in figure 12.

Finally, figure 13 shows the telescopic intramedullary nail 1 engaged into the long bone 100 at the end of the described operations.

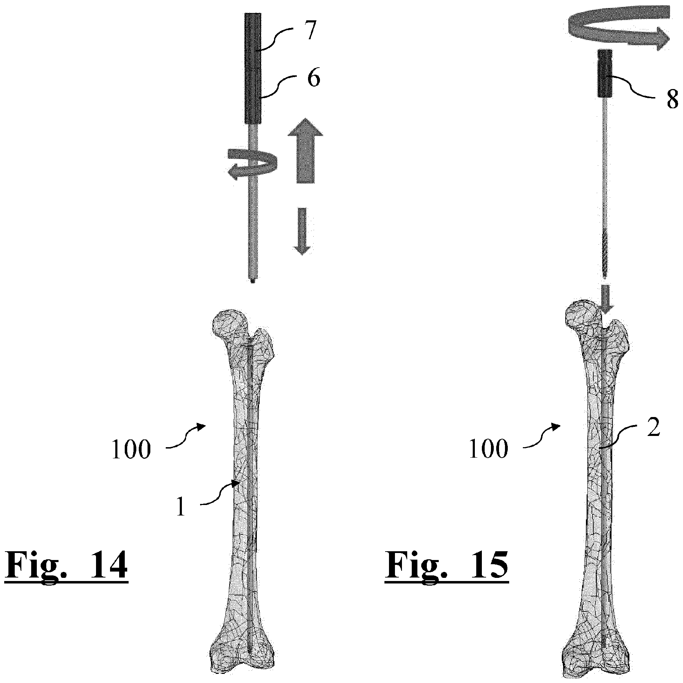

With reference to figures 14 and 15, a procedure for removing the telescopic intramedullary nail 1 is now described.

In a first step, illustrated in figure 14, the rod 3 is extracted by means of the insertion tool 6, 7 previously used for the insertion thereof.

In a second step, illustrated in figure 15, by means of the removal tool 8 a boring is performed up to reach the proximal end of the hollow stem and continue the counterclockwise rotation to couple the removal tool 8 to the component and to unscrew the component from the distal epiphysis 102.

From the previous description it is evident that the telescopic intramedullary nail and the tools dedicated thereto achieve the intended objects and obtain several advantages with respect to the devices of the prior art.

Obviously, in order to meet contingent and specific requirements, a person skilled in the art will be allowed to bring several modifications and alternatives to the above-described invention, all however falling within the scope of protection of the invention as defined by the following claims.

## Claims

1. A telescopic intramedullary nail (1) for the treatment of fractures or deformations of long bones (100), such as for example in the case of osteogenesis imperfecta or pseudoarthrosis, comprising:
a hollow stem (2); and
a rod (3) telescopically coupled inside said hollow stem (2);
wherein said hollow stem (2) comprises, at a free distal end (11), an anchor tip (21; 21') configured to be internally fixed inside a distal portion (102) of a long bone (100) of a patient, without protruding from said long bone (100);
wherein said rod (3) comprises a free proximal end (12) arranged to be fixed to a proximal site of said long bone (100).

2. The telescopic intramedullary nail (1) according to claim 1, wherein said rod (3) comprises, at the proximal end (12), a terminal head (31) arranged to externally abut against a proximal cortical of said long bone (100).

3. The telescopic intramedullary nail (1) according to claim 2, wherein said terminal head (31) is integrally formed with the rest of the rod (3).

4. The telescopic intramedullary nail (1) according to one of claims 2 or 3, wherein said rod (3) comprises, in a position which is adjacent to the terminal head (31), a cylindrical portion (36) with a diameter which is greater than an internal diameter of the hollow stem (2).

5. The telescopic intramedullary nail (1) according to one of claims 2 or 3, wherein said terminal head (31) comprises a conical thread (32) which connects to a cylindrical thread (36') provided on a first proximal section of the rod (3).

6. The telescopic intramedullary nail (1) according to one of the previous claims, wherein said hollow stem (2) comprises, at a proximal end thereof, coupling means (25) for an insertion tool (5, 6) and/or for an extraction tool (8) of said hollow stem (2).

7. The telescopic intramedullary nail (1) according to claim 6, wherein said coupling means (25) are arranged at an insertion mouth of the rod (3) and comprise an internal thread (26) in said insertion mouth.

8. The telescopic intramedullary nail (1) according to claim 7, wherein said coupling means (25) further comprise an end crenelation (27) made frontally to said insertion mouth.

9. The telescopic intramedullary nail (1) according to one of the previous claims, wherein said anchor tip (21) has a thread (22) for the screwing in the epiphysis.

10. The telescopic intramedullary nail (1) according to claims 7 and 9, wherein the screwing direction of the thread (22) of the anchor tip (21) is opposite to that of the internal thread (26) of the coupling means (25) of the hollow stem (5).

11. The telescopic intramedullary nail (1) according to one of claims 1-8, wherein said anchor tip (21') is not threaded but has a transverse through hole (24) for the insertion of an anchor pin.

12. The telescopic intramedullary nail (1) according to one of the previous claims, wherein said anchor tip (21; 21') is cannulated.

13. A kit comprising a telescopic intramedullary nail (1) according to one of the previous claims and at least one removal tool (8) comprising a stem (85) and a boring portion (81) arranged to be coupled, by means of a threaded tip (82) with a left-handed thread, to coupling means (25) arranged at an end of the hollow stem (2).

14. A kit comprising a telescopic intramedullary nail (1) according to one of the previous claims and a drill bit (4) comprising a stem (42) provided with progressive length indexes (40) in order to identify during a boring step the length of the components of the telescopic intramedullary nail (1) to be implanted.

15. A kit comprising a telescopic intramedullary nail (1) according to one of the previous claims; a left-handed tension rod (5) comprising a stem (52) provided with a left-handed threaded tip (51); and a right-handed tension rod (7) comprising a stem (72) provided with a right-handed threaded tip (71).
